# FASCICULE DE BREVET EUROPEEN

(11) **EP 0 681 621 B1**
(45) Date de publication et mention de la délivrance du brevet: **07.01.1998**
(21) Numéro de dépôt: 94905156.9
(22) Date de dépôt: 26.01.1994
(51) Int. Cl.: D04H 13/00, D04H 1/70, A61F 13/00

(54) **NAPPE DE COTON HYDROPHILE ET PRODUITS OBTENUS A PARTIR DE LA TRANSFORMATION D'UNE TELLE NAPPE**
WASSERAUFSAUGENDER BAUMWOLLVLIESSTOFF UND AUS DER TRANSFORMATION DES VLIESSTOFFS HERGESTELLTE PRODUKTE
HYDROPHILIC COTTON LAP AND PRODUCTS OBTAINED BY PROCESSING SAME

(30) Priorité: 29.01.1993 FR 9300928; 05.04.1993 FR 9303964
(43) Date de publication de la demande: 15.11.1995
(73) Titulaire: JAMES RIVER, 68320 Kunheim (FR)
(72) Inventeur: NEVEU, Jean-Louis, F-27690 Léry (FR)
(74) Mandataire: David, Daniel
(86) Numéro de dépôt international: FR9400091
(87) Numéro de publication internationale: WO9417235

(56) Documents cités:
- EP-A- 0 295 038
- EP-A- 0 405 043
- FR-A- 2 258 188
- US-A- 3 589 367

## Description

L'invention concerne généralement une nappe de coton hydrophile et les nouveaux produits obtenus à partir de la transformation de cette nappe, en particulier de sa découpe.

Les nappes selon l'invention, constituées à cent pour cent de fibres de coton, sont utilisées pour préparer des produits découpés ou prédécoupés sous forme paquetée c'est-à-dire sous la forme d'une bande pliée en accordéon et emballée dans un sachet, que l'on extrait à la demande. Ce type de produit est destiné principalement aux soins dermatologiques et chirurgicaux. La nappe peut être aussi découpée sous la forme de tampons ou formats individualisés du type disques ou rondelles, ou encore carrés, destinés aux soins du visage tels que le démaquillage, ou à la toilette des bébés. Par ailleurs, des produits composites cellulosiques préparés à partir de la nappe selon l'invention et découpés, peuvent être utilisés par exemple comme tampons oculaires ou cosmétiques ou encore comme compresses.

De nombreux produits à base de coton hydrophile existent actuellement sur le marché.

La Demanderesse commercialise elle-même des produits de coton hydrophile sous la marque enregistrée LOTUS pour du coton paqueté, prédécoupé ou en formats.

Pour les produits de coton sous forme paquetée, le procédé usuel de fabrication de la nappe consiste à soumettre du coton écru en balles, à des opérations de nettoyage et d'ouvraison, à un traitement en "batch", consistant à enlever les graisses, les cires, au moyen d'une solution de débouillissage à base de soude, pour rendre le coton hydrophile, suivi d'un rinçage et exprimage, puis à un traitement de blanchiment, toujours en "batch", au moyen par exemple d'eau oxygénée ou peroxyde d'hydrogène. Le coton blanchi est le cas échéant traité au moyen de lubrifiants et/ou d'adoucissants (ensimages). Le coton est séché avant d'être transformé par des cardes qui alignent les fibres pour former des voiles. On forme la nappe en associant plusieurs voiles de carde, préalablement pliés dans le sens de production pour avoir la largeur désirée. Enfin, on plie la nappe en accordéon pour être empaquetée.

Cette nappe peut être éventuellement prédécoupée avant conditionnement.

Le produit obtenu est volumineux. Les nappes ont un grammage d'environ 400 à 600 g/m². Les emballages sont de grande taille ce qui entraîne des problèmes de stockage et de présentation dans les rayonnages lors de leur mise en vente.

Le produit de coton ainsi fabriqué a un aspect très pelucheux et s'effiloche facilement. De plus, la nappe prédécoupée ou découpée se délamine facilement, les voiles de carde se désolidarisent les uns des autres, notamment dans le cas de coton non prédécoupé.

En ce qui concerne les produits de coton en formats, le procédé de traitement des fibres est identique à celui décrit ci-dessus (traitement chimique et séchage). Puis on mélange à ce coton traité, des fibres synthétiques dans un rapport fibres synthétiques-fibres de coton d'environ 5:95 à environ 30:70. Les fibres synthétiques sont des fibres thermofusibles, par exemple de polyéthylène, de polypropylène, ou des fibres bicomposantes. Elles sont incorporées aux fibres de coton dans des mélangeuses ou ouvreuses-mélangeuses. La nappe est ensuite formée sur un nappeur pneumatique et/ou sur des cardes. Elle passe dans un four du type à "air chaud traversant" dont la température est suffisante pour fondre les fibres synthétiques. Lorsqu'elles fondent, ces fibres forment un liant entre les fibres de coton et procurent à la nappe après refroidissement une cohésion. Après refroidissement de la nappe, celle-ci est découpée pour réaliser des formats.

Un autre procédé de fabrication d'une nappe destinée à des produits en formats est décrit dans la demande de brevet français n° 2 552 120.

Ce procédé consiste à effectuer le traitement chimique de débouillissage et de blanchiment sur une nappe écrue déjà formée, enroulée sur un cylindre creux comportant des perforations sur sa surface, le cylindre étant ensuite placé dans un corset. On fait circuler les liquides de traitement radialement au travers des spires de la bobine formée par la nappe dans un autoclave, de manière telle qu'il s'établisse une pression différentielle entre le liquide entrant dans la bobine et le liquide en sortant. Cette pression différentielle a pour conséquence d'augmenter la cohésion de la nappe. Celle-ci est ensuite rincée. Puis elle est découpée en formats sous forme de rondelles.

Au vu de ce qui précède, les nappes de coton sont fabriquées de manière différente en fonction de leur utilisation : produit paqueté ou en formats.

La demande de brevet européen n° 0 405 043 décrit des disques de coton comprenant une couche centrale et au moins une couche externe préalablement comprimée, chaque couche correspondant à une nappe de coton déjà blanchie.

Les couches formant le disque sont liées les unes aux autres, après découpe, uniquement par leurs bords, lorsque le disque n'a pas subi de compression à l'état fini.

Ce produit non comprimé à l'état fini, ne présente pas une bonne cohésion de surface, les couches n'adhérant pas entre elles.

Par ailleurs, les produits de coton hydrophile, actuellement sur le marché, ne sont généralement pas entièrement satisfaisants pour les consommatrices qui recherchent un produit très doux, ayant une excellente tenue, résistant à la délamination et ayant un excellent état de surface.

La présente invention a pour but de pallier l'ensemble des inconvénients précités en fournissant une nappe de coton hydrophile, constituée à cent pour cent de fibres de coton, qui présente une excellente cohésion sans traitement de compression et une très bonne résistance à la délamination.

L'invention a de plus pour objet une nappe de coton hydrophile, qui tout en présentant une excellente cohésion, reste très douce au toucher.

L'invention a encore pour objet une nappe de coton hydrophile, qui a un très bel aspect et état de surface, ne s'effiloche pas en surface et présente une résistance au peluchage nettement améliorée par rapport aux produits de l'art antérieur.

L'invention a également pour objet une nappe de coton hydrophile compacte, permettant un gain considérable de volume pour l'emballage et le stockage.

En outre, l'invention a pour objet une nappe de coton hydrophile utilisable à la fois pour des produits paquetés et des produits en formats, sans traitement supplémentaire de la nappe autre que la découpe ou la prédécoupe.

L'invention a par ailleurs pour objet les nouveaux produits de coton obtenus par découpe ou prédécoupe de la nappe.

L'invention a encore pour objet des nouveaux produits composites cellulosiques découpés, à base de coton hydrophile, comprenant la nappe constituée à cent pour cent de fibres de coton.

Selon l'invention, la nappe de coton hydrophile est caractérisée en ce que les couches extérieures (3) et (4) entourant la couche centrale (2) sont des voiles de corde, en ce que la couche centrale (2) comprend des fibres de coton stratifiées orientées de manière sensiblement oblique entre les deux plans formés par les couches extérieures et en ce que la nappe (1) est obtenue par un procédé en ligne continue, consistant essentiellement à superposer les trois couches et à leur faire subir un traitement de débouillissage, un traitement de blanchiment puis un rinçage, conférant aux couches une très bonne cohésion entre elles.

Selon une caractéristique avantageuse de l'invention, les voiles de carde précités sont sensiblement isotropes et obtenus de préférence au moyen d'une carde de type pêle-mêle, connue en soi.

Selon une autre caractéristique de l'invention, chaque couche extérieure a un grammage compris dans l'intervalle allant d'environ 5 à environ 80 g/m², et de préférence d'environ 15 à environ 30 g/m².

Selon encore une autre caractéristique de l'invention, la couche centrale a un grammage compris dans l'intervalle allant d'environ 60 à environ 400 g/m².

D'autres caractéristiques et avantages de l'invention apparaîtront plus clairement à la lecture détaillée de la description qui suit en référence aux dessins annexés dans lesquels :
- la figure 1 représente une coupe transversale schématique de la nappe de coton hydrophile selon l'invention,
- la figure 2 représente schématiquement une coupe transversale d'un produit de coton selon l'invention, découpé, tel qu'une rondelle,
- la figure 3 est une série de carrés de coton prédécoupés reliés par des points d'attache,
- les figures 4a et 4b sont des microphotographies du bord d'une rondelle de coton selon l'invention, à un grossissement respectivement de 20 et 200 fois,
- les figures 5a et 5b sont des microphotographies d'une coupe transversale d'une rondelle selon l'invention, au niveau respectivement de chacune des faces de la rondelle à un grossissement de 66 fois,
- la figure 6 est une microphotographie d'une des surfaces de la rondelle selon l'invention, à un grossissement de 200 fois,
- les figures 7a et 7b sont des microphotographies du bord d'une rondelle témoin B cent pour cent coton, à un grossissement respectivement de 20 et 200 fois,
- les figures 8a et 8b sont des microphotographies d'une coupe transversale d'une rondelle témoin B, au niveau de chacune de ses faces, à un grossissement respectivement de 100 et 66 fois ;
- la figure 9 est une microphotographie d'une surface d'une rondelle témoin B, à un grossissement de 200 fois ;
- la figure 10 est une microphotographie d'une coupe transversale d'une autre rondelle témoin C obtenue à partir d'une nappe comprenant des fibres synthétiques, à un grossissement de 66 fois ;
- la figure 11 est une microphotographie d'une surface d'une rondelle témoin C, à un grossissement de 200 fois ;
- la figure 12 représente une coupe transversale schématique d'un produit composite en formats selon l'invention, découpé sous forme de rondelle ; et
- la figure 13 représente une coupe transversale schématique d'un autre produit composite en formats selon l'invention, découpé sous forme de rondelle.

La nappe selon l'invention est fabriquée suivant le procédé en continu décrit ci-après.

Un mélange de fibres de coton de diverses origines et qualités subit un traitement d'ouvraison et de nettoyage. On forme une nappe de grammage compris entre 100 et 600 g/m² par tout moyen approprié mécanique, tel qu'une carde, et/ou un nappeur pneumatique. Dans le cas présent, on superpose trois couches.

La première couche est constituée d'un voile de carde obtenu par exemple au moyen d'une carde de coton classique, et de préférence au moyen d'une carde de type pêle-mêle connue en soi. La carde de type pêle-mêle est une machine mécanique qui se distingue par la sortie du voile. qui permet aux fibres de n'être plus parallélisées dans le sens de production (voiles de fibres brouillées). Le terme carde de type pêle-mêle englobe ici aussi bien les cardes pêle-mêle classiques que des cardes pêle-mêle munies d'une sortie pneumatique, comme par exemple des cardes pêle-mêles commercialisées par Fehrer.

La seconde couche est obtenue par voie pneumatique, notamment sur une machine du type Rando. Au moyen de cette dernière, les fibres sont projetées par un tambour garni de dents dans un courant d'air et sont aspirées sur un tapis ou tambour au travers duquel on crée une dépression. Les fibres sont réparties au hasard dans toutes les directions.

Dans la pratique de l'invention, on observe une structure de type stratifié pour la couche sortie de cette machine. Les fibres de la couche déposée par voie pneumatique sont en effet orientées de manière sensiblement oblique par rapport aux plans inférieur et supérieur horizontaux de la nappe.

La troisième couche est constituée d'un voile de carde similaire à la première couche. L'ensemble forme ainsi une nappe de type complexe comprenant trois couches superposées (nappe "sandwich").

Cette nappe est ensuite entraînée par un tapis support sans fin, perméable aux liquides, vers les différents postes de traitement en ligne continue. On imprègne la nappe en déversant par gravité une solution de débouillissage contenant de la soude, sur la nappe, sous la forme d'une lame liquide transversale à la direction de déplacement de celle-ci. On crée, au moyen d'une fente d'aspiration disposée sous la toile, une dépression suffisante pour permettre à au moins une partie de la solution de traverser la nappe. On contrôle en même temps la quantité de liqueur retenue par la nappe en réglant le vide créé au niveau de la fente aspirante. On introduit la nappe dans un vaporiseur chauffé à une température voisine de 100°C dans lequel elle séjourne, tout en restant continue, pendant un temps donné notamment en fonction du débit matière (kg/h de coton).

On rince ensuite la nappe, on extrait le jus de débouillissage au moyen d'une deuxième lame liquide et d'une fente à vide associée à un vide moyen.

On imprègne la nappe débouillie hydrophile, avec une solution de blanchiment contenant par exemple du chlorite, de l'hypochlorite de sodium ou de préférence de l'eau oxygénée, de la même manière que pour le traitement de débouillissage. On introduit ensuite la nappe dans un vaporiseur chauffé à une température d'environ 100°C pour que le blanchiment soit effectif.

On rince ensuite la nappe au moyen d'une succession de lames liquides associées à des fentes aspirantes.

Ce traitement de la nappe confère une adhérence entre les couches la constituant et une très bonne cohésion à l'ensemble, sans aucun traitement mécanique ou chimique supplémentaire. par exemple de compression.

Après séchage, cette nappe peut être utilisée directement pour la préparation de coton hydrophile paqueté ou en formats.

Cette étape de transformation implique une opération de découpe réalisée au moyen d'une presse de type alternatif ou d'un outil rotatif.

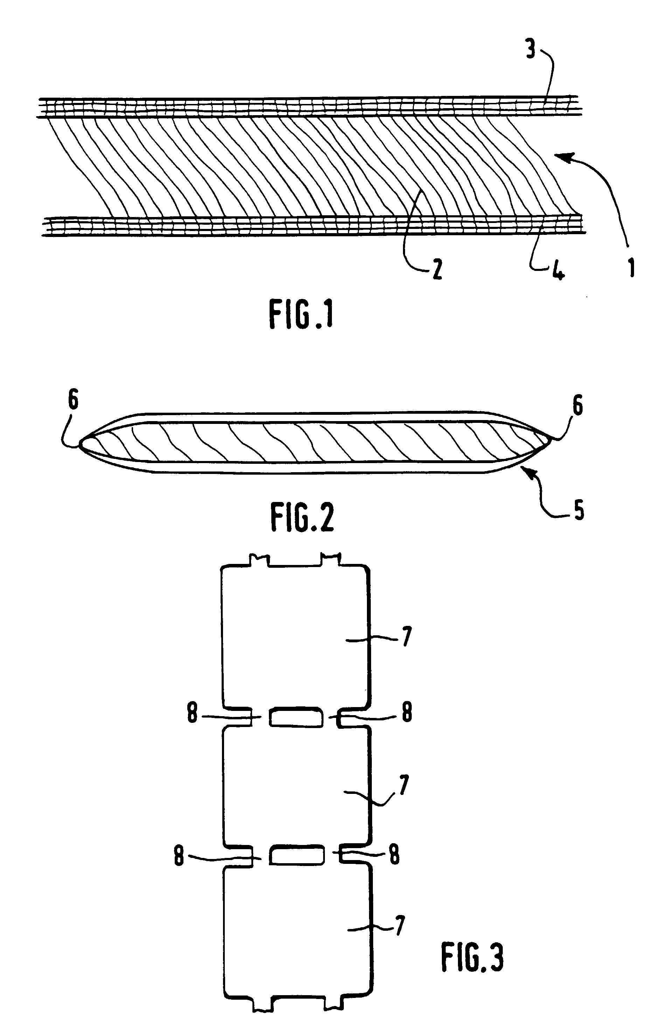

Comme l'illustre la figure 1, la nappe de coton hydrophile 1, cent pour cent coton, se compose de trois couches : une couche centrale 2 et deux couches extérieures ou de surface 3 et 4 entourant la couche centrale 2.

La couche centrale 2 est la plus épaisse. Elle a un grammage variant dans l'intervalle allant d'environ 60 à environ 400 g/m². Lorsque le grammage de la couche centrale est situé vers la limite inférieure de l'intervalle, par exemple 150 g/m², la nappe très compacte est utilisée de préférence pour un produit de coton du type en formats. Lorsque le grammage de cette couche centrale tend vers la limite supérieure de l'intervalle, la nappe est utilisée de préférence pour un produit de coton de type paqueté, plus volumineux. Cette couche centrale est caractérisée par ses fibres de coton stratifiées, orientées de manière sensiblement oblique entre les deux plans formés par les couches latérales. Cette orientation des fibres découle de la fabrication de cette couche centrale par voie pneumatique, au moyen d'une machine de type Rando.

Les couches extérieures ou de surface 3 et 4 sont des voiles de cardes obtenus au moyen d'une carde du type pêle-mêle. Chaque couche extérieure a un grammage compris dans l'intervalle allant d'environ 5 à environ 80 g/m² et de préférence d'environ 15 à environ 30 g/m². Ces voiles sont bidimensionnels et sensiblement isotropes dans le plan de leur formation. Ils comprennent des fibres orientées à la fois dans le sens marche et dans le sens travers. Les fibres de coton pour les voiles de carde, sont choisies parmi les belles et longues fibres. Les couches 3 et 4 confèrent à la nappe un état de surface exceptionnel. En effet, la nappe ne peluche pas, ne s'effiloche pas non plus, comparativement aux produits cent pour cent coton actuellement distribués sur le marché, du fait de l'orientation des fibres de surface qui sont disposées dans deux directions orthogonales ou de manière isotrope. Les voiles de carde forment un écran, masquent les fibres de la couche centrale et retiennent ces dernières. De ce fait, lors de leur utilisation, par exemple sur la peau, les produits de coton hydrophile selon l'invention, ne se désagrègent pas et ne laissent pas de fibres adhérant sur la peau.

La nappe est ensuite découpée ou prédécoupée sous la forme d'une rondelle ou disque, d'un carré, d'un rectangle ou tout autre polygone régulier. Les produits de coton ainsi obtenus à la différence des produits découpés de l'art antérieur, ont la particularité d'être sertis au cours de cette étape de découpage qui est effectuée dans une machine de découpe à l'emporte-pièce, une machine de découpe mécanique de type alternatif ou rotatif ou tout autre machine conduisant à un rapprochement des couches entre elles dans la zone de la coupe.

L'expression "machine de type alternatif' sera utilisée dans la description qui suit pour désigner une machine ou un outil de découpe mécanique de type alternatif.

De même, l'expression "machine de type rotatif" sera utilisée dans la description qui suit pour désigner une machine ou un outil de découpe mécanique de type rotatif.

Comme l'illustre la figure 2, le produit de coton découpé, ici la rondelle 5, comporte des bords 6 d'épaisseur réduite d'environ 0,2 millimètre, alors que l'épaisseur au centre de la rondelle est de 1,5 millimètre ou plus. Le produit de coton découpé comporte à sa périphérie des fibres liées mécaniquement par des forces de compression ou d'écrasement exercées lors de la coupe. Le produit serti comporte une enveloppe scellée correspondant aux deux couches 3 et 4 de surface, liées entre elles. Cette cohésion périphérique renforce la cohésion déjà existante de surface de la couche centrale 2 avec chacune des couches extérieures 3 et 4. Un tel produit serti peut s'utiliser sur la tranche sans risque de délamination et offre un aspect plus net.

Il est à noter que l'obtention de la nappe selon l'invention ne nécessite pas de traitement d'ensimage.

Les produits paquetés ou en formats peuvent être prédécoupés de manière à laisser des points de fixation entre les différents formats ou rectangles d'une bande de coton paqueté. Ce nouveau produit est illustré à la figure 3. Les différents formats, ici carrés, sont reliés entre eux par des points de fixation 8 facilement déchirables. Les formats reliés entre eux peuvent être empaquetés sous forme de bandes pliées en accordéon. Ce produit est de fabrication plus simple et plus rapide que les produits en formats.

La nouvelle nappe de coton selon l'invention présente de nombreux avantages dont certaines caractéristiques tout à fait inattendues.

On mesure certaines propriétés de la nappe ou des produits obtenus à partir de la transformation de cette nappe, et on les compare aux propriétés de produits de l'art antérieur.

Plus précisément, on prépare d'une part, une nappe selon l'invention (I) blanchie et découpée en bandes, et d'autre part une nappe témoin. Cette nappe témoin (A) est une nappe cent pour cent coton, où les fibres de coton sont traitées chimiquement de manière classique. Elle est obtenue par cardage de bourre.

Ces différentes nappes : nappe selon l'invention (I), nappe témoin (A) sont prédécoupées en formats de 50 millimètres sur 100 millimètres, reliés entre eux par des points d'attache.

On utilise également des rondelles cent pour cent coton que l'on trouve sur le marché. Ces rondelles seront référencées ci-après témoin B.

Pour chacun des tests décrits ci-après, on effectue les mesures sur 5 échantillons de produit (nappe, formats ou rondelles) selon l'invention et sur 5 échantillons de produit témoin. Les valeurs mentionnées pour les différents tests sont les moyennes des mesures.

### Caractéristiques de la nappe.

On mesure pour chaque nappe :
- le grammage,
- l'épaisseur sous une pression de 20 g/m² exercée sur la nappe, et
- le bouffant correspondant à l'épaisseur de la nappe sans pression notable exercée sur la nappe (en toute rigueur une pression très faible de 2 g/m² est appliquée).

On calcule la densité apparente par le rapport du grammage sur le bouffant.

Les résultats sont rapportés dans le tableau ci-après :

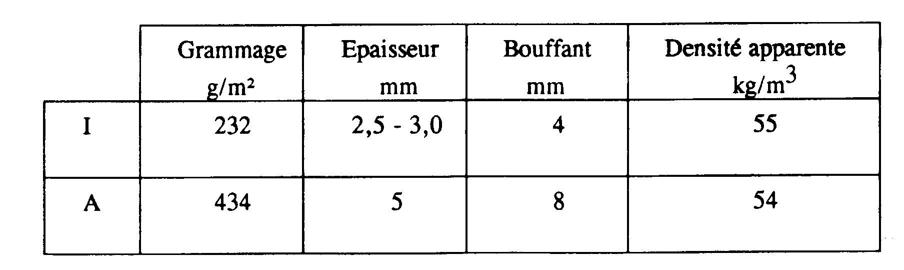

Ce tableau montre que les densités apparentes des deux types de nappes sont quasiment identiques.

### Densité apparente des rondelles.

La densité apparente est mesurée d'une part pour des rondelles obtenues par découpe de la nappe selon l'invention sur machine de type alternatif et d'autre part pour des rondelles témoins B.

Les valeurs rapportées dans le tableau ci-après sont données pour un grammage de rondelle équivalent.

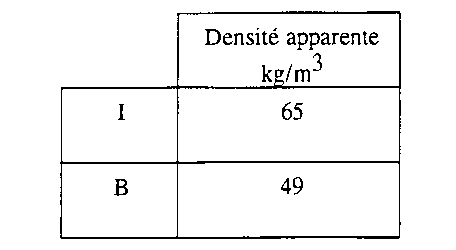

Ceci montre que les produits découpés selon l'invention, du type rondelle, sont beaucoup plus compacts que les produits témoins B. Ceci peut s'expliquer par le sertissage qui renforce le compactage du produit, par rapport aux produits classiques qui ne sont pas sertis, en l'occurrence ici le produit témoin B.

Ces produits plus compacts sont bien appréciés des utilisatrices.

Par ailleurs, comme la nappe est très compacte et sa densité augmentant (à masse identique, le volume de la nappe diminue), les produits obtenus sont beaucoup moins volumineux que les produits classiques. En conséquence, le gain de place dans les emballages comme dans le stockage et dans les rayonnages, est considérable. La réduction de volume est d'environ 25 % ou plus. Les paquets de coton diminuent ainsi par exemple en hauteur.

### Séparation des formats prédécoupés

Les formats prédécoupés sur machine de type alternatif ou rotatif sont reliés entre eux par des points d'attache. On mesure ici la force nécessaire pour séparer deux formats l'un de l'autre, c'est-à-dire pour rompre les points d'attache. Cette force est exprimée en Newton.

Le test est réalisé sur un dynanomètre, plus précisément un appareil commercialisé sous la marque Instron. Les caractéristiques pour ce test sont les suivantes :
- vitesse de l'Instron : 100 mm/min
- Ecart entre les mâchoires de l'Instron : 50 mm
- Largeur de l'éprouvette : 95 mm.

Les résultats sont résumés dans le tableau ci-après.

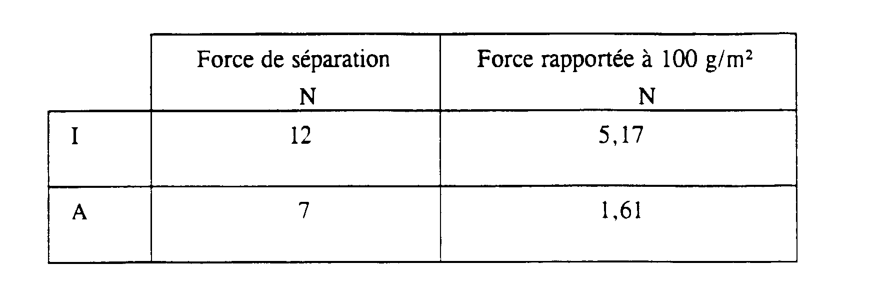

On peut donc observer que la force nécessaire pour séparer deux formats est beaucoup plus grande dans le cas des formats obtenus à partir de la nappe selon l'invention bien que cette dernière soit beaucoup plus légère.

Cette caractéristique est tout à fait inattendue. Le produit est alors plus résistant à la déformation. On peut détacher les formats les uns des autres en exerçant une force de traction plus énergique, tout en gardant un produit (un format) non déformé, prêt à être utilisé.

### Résistance à la rupture des nappes

On mesure la résistance à la rupture (Rr) exprimée en Newton de chaque nappe dans le sens marche et dans le sens travers sur un appareil commercialisé sous la marque Instron, en tirant sur la nappe jusqu'à la rupture de celle-ci. La résistance à la rupture (Rr) est exprimée en Newton.

Les caractéristiques pour ce test sont les suivantes :
- Vitesse de l'Instron : 100 mm/min
- Largeur éprouvette : 100 mm
- Ecart entre les mâchoires de l'Instron : 25 mm

Les résultats sont rapportés ci-après :

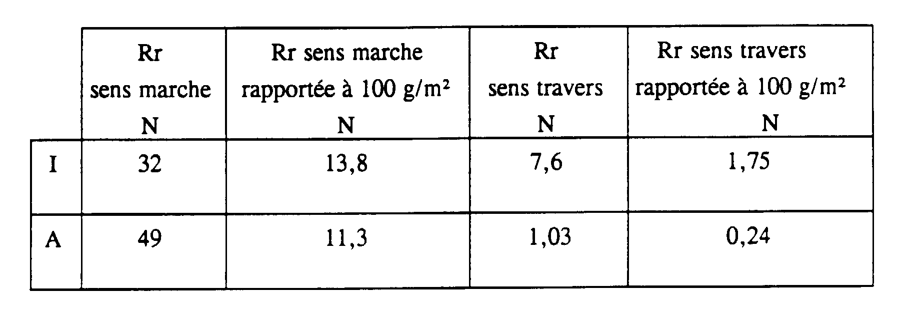

On peut noter à partir des valeurs du tableau ci-dessus, que la résistance à la rupture dans le sens marche, ramenée à 100 g/m², est de manière surprenante plus grande pour la nappe selon l'invention. L'augmentation de la résistance dans le sens travers est encore plus marquée.

Ceci se traduit par un produit plus solide, ayant une meilleure tenue.

La résistance à la rupture dans le sens travers peut s'expliquer par le procédé utilisé dans la présente invention. En effet, la nappe étant formée de manière pneumatique sur une machine de type Rando, les fibres sont orientées dans deux et même trois dimensions. Certaines fibres sont orientées dans une direction diagonale par rapport au sens marche.

Les procédés utilisés pour fabriquer les nappes de l'art antérieur, forment des nappes où les fibres sont parallèles et ne présentent aucun moyen de liaison entre elles. Par conséquent, ces nappes ont une très mauvaise résistance à la rupture dans le sens travers.

### Délamination de la nappe et des produits selon l'invention

Dans ce test, on compare la résistance à la délamination d'une nappe ni découpée ni prédécoupée, avec un produit découpé, plus précisément une rondelle, sur une machine de type alternatif. Ces produits se distinguent l'un de l'autre par le fait que la nappe présente des bords qui ne sont pas sertis, et les rondelles présentent un contour serti, comme l'illustre la figure 2, résultant de la découpe.

Le test est réalisé sur un appareil Instron. Les caractéristiques du test sont les suivantes :
- Vitesse de l'Instron : 20 mm/min
- Largeur de l'éprouvette : 50 mm
- Ecartement entre les mâchoires : 50 mm

Les nappes utilisées ont un grammage de 200 g/m².

Les valeurs de résistance à la délamination exprimées en centiNewton sont données ci-après :

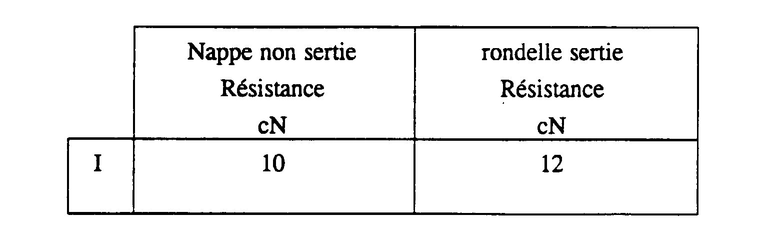

Il est à noter que la nappe avant tout sertissage présente déjà une bonne résistance à la délamination grâce à la liaison entre les couches résultant de son procédé de fabrication en continu.

Au vu des mesures exposées ci-dessus, le sertissage améliore encore la résistance à la délamination.

Ce phénomène d'association par sertissage n'apparaît, dans le cas de la nappe selon l'invention, que lorsqu'elle n'a été soumise à aucun traitement d'ensimage, et procure des effets inattendus.

Ainsi, la nappe de coton en raison de la cohésion intercouches obtenue par les traitements physique et chimique spécifiques de son procédé de fabrication et du sertissage résultant de la découpe, permet de fournir des produits dont la résistance à la délamination est très surprenante. Des rondelles de coton de ce type, empaquetées dans un sachet, s'individualisent très bien lorsqu'elles sont extraites du sachet. Et lors de leur utilisation, elles ne se séparent pas en plusieurs couches comme le font les produits classiques.

Malgré une cohésion importante et une grande compacité, ces produits gardent une très grande douceur d'utilisation.

### Autres caractéristiques

Dans la nappe et les produits selon l'invention, l'équilibre absorption-résistance à l'arrachement est tout à fait satisfaisant. Le nombre de noeuds (neps) dans la nappe selon l'invention est faible comparé aux produits connus jusqu'alors.

### Résistance au peluchage

On mesure par le test du "Pilling", le peluchage des produits selon l'invention en comparaison avec les produits actuellement sur le marché.

Le test est réalisé sur des rondelles découpées.

Il consiste à poser ou appliquer un matériau adhésif sur la surface de la rondelle, à retirer ce matériau adhésif, et à comparer la quantité de fibres retirées du produit.

Le produit selon l'invention peluche peu comparativement aux autres.

Cette caractéristique peut être reliée à la caractéristique de densité apparente du produit, qui est plus grande dans le cas de l'invention.

Ceci contribue à améliorer l'aspect visuel du produit qui est excellent. Le fini et la tenue du produit sont également largement appréciés lors de l'utilisation des produits.

Certaines caractéristiques de la nappe et des produits obtenus à partir de la transformation de celle-ci, mentionnées dans le texte qui précède, ont également pu être observées et comparées avec des produits de l'art antérieur, à partir des microphotographies en annexe.

Les produits de l'art antérieur sont illustrés ci-après, d'une part, par les rondelles témoins B, cent pour cent coton, et d'autre part, par des rondelles témoins C obtenues à partir d'une nappe de coton comprenant des fibres synthétiques liantes apportant une certaine cohésion à la nappe.

Les différentes photos de rondelles ont été prises en microscopie électronique.

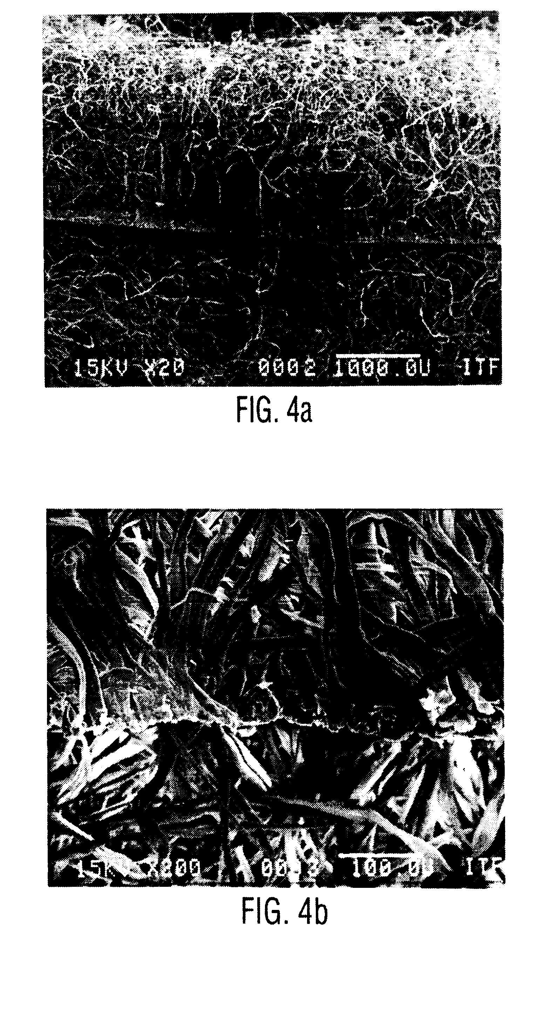

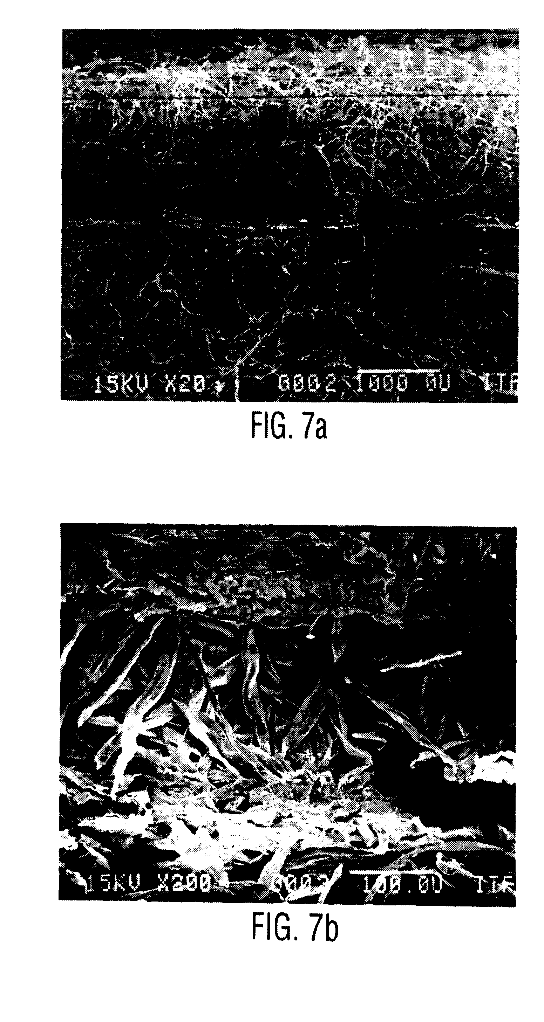

Les figures 4a-4b et 7a-7b correspondent à des vues du bord de chacune des rondelles.

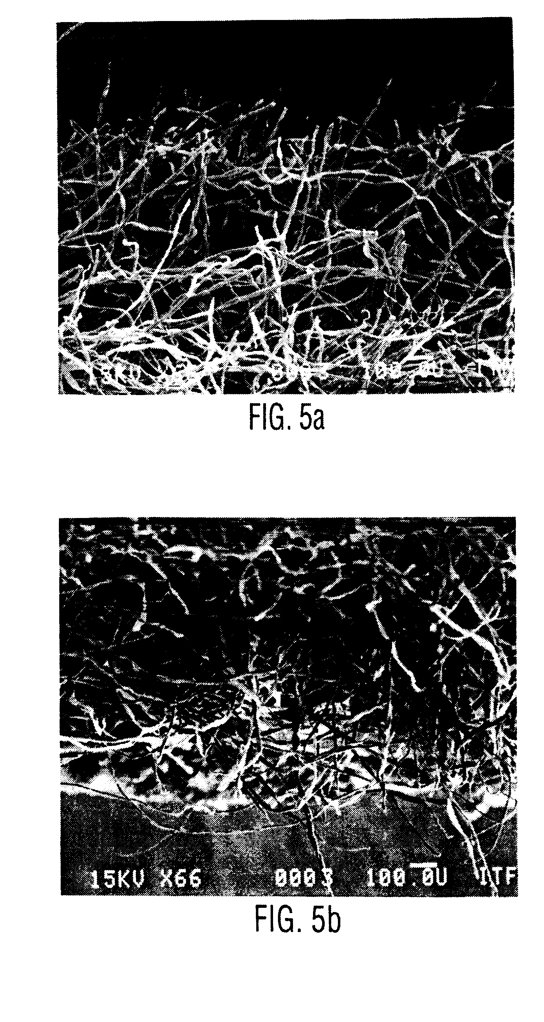

Les figures 5a-5b, 8a-8b et 10 correspondent à des coupes transversales réalisées dans la partie centrale de chaque rondelle.

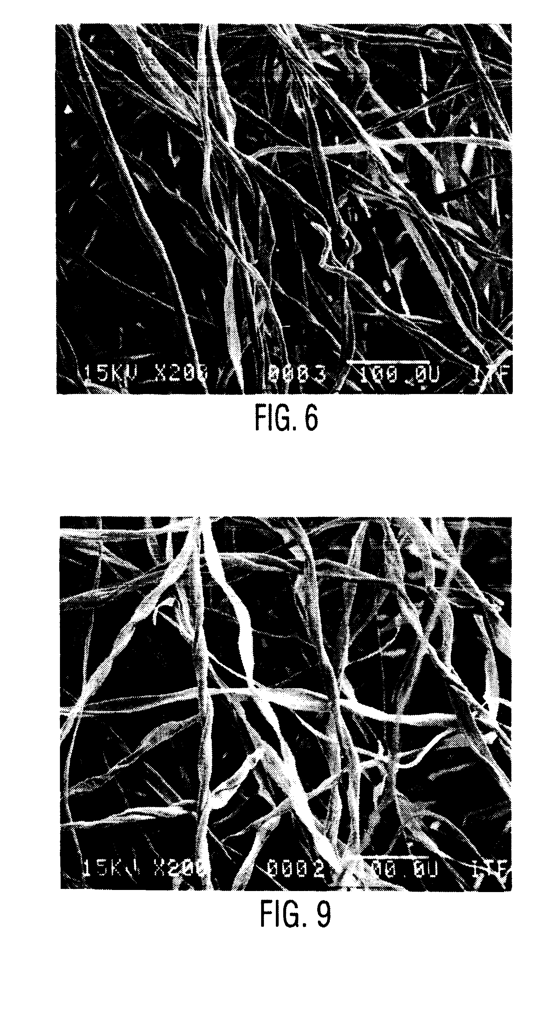

Et les figures 6, 9 et 11 correspondent à des vues de surface des rondelles.

La photo de la figure 4a montre clairement une ligne nette horizontale de sertissage obtenue par découpe sur une machine de type alternatif. Les produits selon l'invention présentent un bord d'épaisseur très réduite et délimité par cette ligne de sertissage.

La photo de la figure 4b montre la soudure des fibres de coton entre elles. Cet effet est obtenu par la découpe de la rondelle sur une machine de type alternatif.

En comparaison, la rondelle témoin B représentée en figure 7a, comporte plusieurs lignes discontinues irrégulières au niveau du bord de la rondelle. Il n'y a pas de ligne unique précise de sertissage. Un effet "mille-feuilles" est obtenu. La photo 7b montre quant à elle que les fibres ne sont pas toutes liées entre elles.

Quant à la rondelle témoin C, aucune photo du bord n'est rapportée puisqu'il n'y a pas de sertissage.

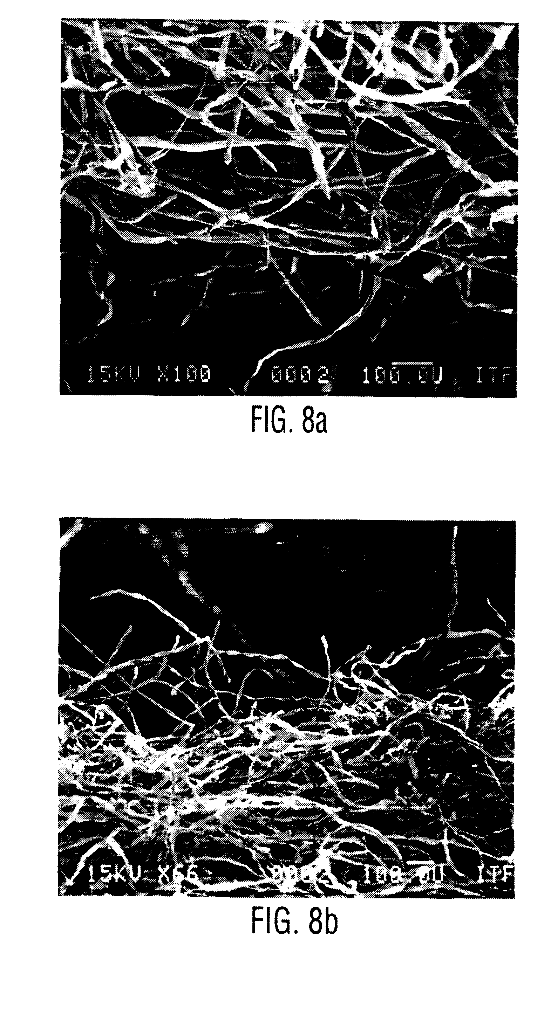

Les figures 5a-5b et 8a-8b correspondent respectivement à des coupes transversales de rondelles de la nappe selon l'invention et de rondelles témoins B. On peut observer que les fibres sont orientées dans trois dimensions. Dans le cas de l'invention, cette orientation des fibres découle de la formation de la nappe sur un nappeur pneumatique. Plus précisément, la figure 5b illustre cette répartition tridimensionnelle des fibres. Par ailleurs, on observe dans la partie inférieure de cette coupe transversale, une zone centrale comportant des fibres apparaissant nettement sur la photo et deux zones plus "floues" de part et d'autre de cette zone centrale où les fibres sont dans un plan situé à l'arrière de celui de la coupe photographiée. Ceci illustre l'effet de quadrillage sur les fibres, provoqué ici sur une face de la rondelle, par le tapis ou toile du séchoir.

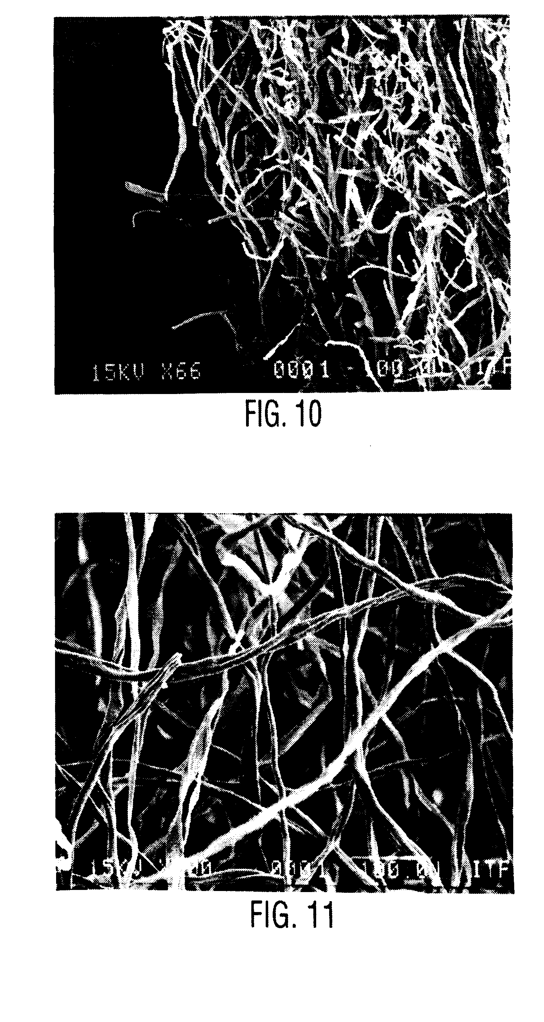

La figure 10 représentant la coupe d'une rondelle témoin C, illustre la répartition bidimensionnelle des fibres, résultant des voiles de carde et de leur superposition dans la nappe.

On peut également observer sur la figure 10, la présence de fibres rondes et lisses qui sont des fibres synthétiques liantes (rondelle témoin C).

Sur la figure 6 qui est une photo de la surface de la rondelle selon l'invention, on peut remarquer la répartition régulière et équilibrée des fibres.

La surface des rondelles témoins B et C illustrées respectivement par les microphotographies des figures 9 et 11 est plus irrégulière. On peut également observer sur la figure 9, la présence de nombreuses fibres de "coton mort" correspondant aux fibres très fines, et sur la figure Il, la présence de fibres synthétiques.

Les rondelles selon l'invention, illustrées par les microphotographies, ont de manière générale un plus bel aspect fini comparativement aux rondelles obtenues à partir des nappes de l'art antérieur.

L'invention comprend également des produits en formats obtenus par découpe ou prédécoupe d'au moins deux nappes selon l'invention telles que décrites précédemment. Ces produits plus épais sont par exemple des rondelles comprenant deux nappes liées l'une à l'autre par leurs bords, par sertissage. Les nappes sont découpées dans ce cas, de préférence sur une machine de type rotatif. qui a pour particularité d'améliorer le sertissage entre les nappes.

L'invention a également pour objet un produit composite en formats à base de coton hydrophile, et comprenant une nappe constituée à cent pour cent de fibres de coton comportant elle-même trois couches, telle que décrite dans la description qui précède, et au moins une quatrième couche externe constituée au moins partiellement de fibres cellulosiques et située sur l'une des faces de la nappe précitée comportant les trois couches. Ce produit composite en formats est serti lors de la découpe sur une machine de découpe rotative. La ou les couches externes supplémentaires ne sont pas ensimées.

Les produits en formats peuvent être découpés sous la forme d'une rondelle, d'un carré, d'un rectangle, ou tout autre polygone régulier.

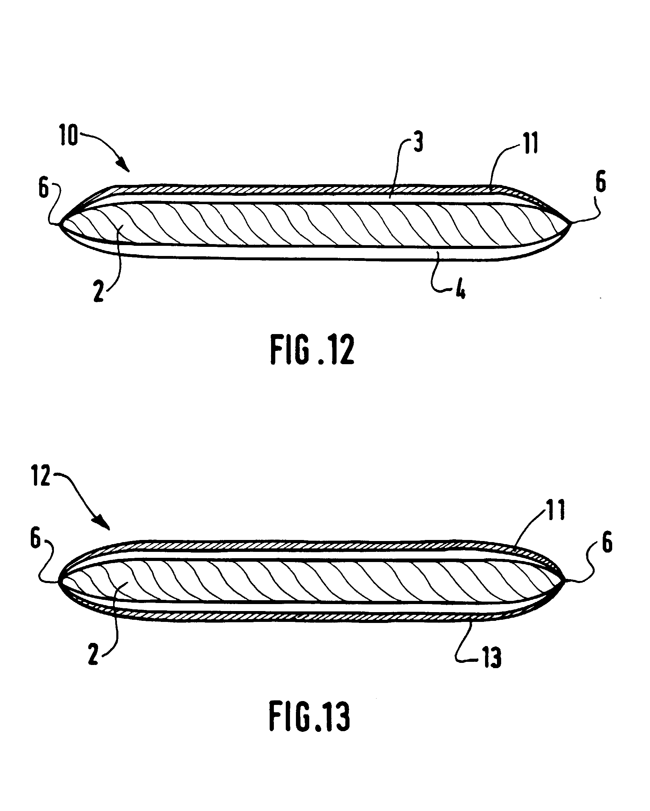

Ce produit composite en formats est illustré par les figures 12 et 13 où il est représenté sous la forme d'un format découpé et plus précisément d'une rondelle.

Suivant la figure 12, le format ou la rondelle 10 comprend la nappe constituée à cent pour cent de fibres de coton, qui est formée de la couche centrale 2 et des deux couches extérieures 3 et 4, et une couche externe non ensimée 11 constituée de fibres cellulosiques et située sur la couche extérieure 3. Le format ou la rondelle composite formé par découpe sur une machine de découpe rotative comporte des bords 6 d'épaisseur réduite. Le sertissage est de manière surprenante excellent.

Suivant la figure 13, le format ou la rondelle 12 comprend de plus une cinquième couche externe 13 située sur la couche extérieure 4 de la nappe constituée à cent pour cent de fibres de coton.

Les couches 11 et 13 peuvent être en matériaux identiques ou différents.

Les fibres cellulosiques constituant au moins partiellement le matériau de ces couches externes sont par exemple des fibres papetières ou encore des fibres textiles en partie d'origine naturelle ou artificielle.

Un produit composite en formats peut donc présenter une face absorbante correspondant à une couche extérieure de la nappe cent pour cent coton hydrophile et une face moins absorbante ou imperméable, de protection, correspondant à la couche externe. Cette dernière est constituée par exemple d'une couche de "papier tissue" ou ouate de cellulose ou encore d'une couche de papier qui a été plastifiée.

La couche externe du produit ou du format composite peut encore être un nontissé lié par adhésif, ou thermolié ou encore lié par jets d'eau. Ce nontissé doit contenir au moins partiellement des fibres textiles, cellulosiques, telles que des fibres de coton, de viscose ou autres.

Ces produits de coton composites en formats peuvent être utilisés pour appliquer des produits cosmétiques ou pharmaceutiques sous forme de crèmes, onguents ou gels sur la peau sans que les doigts maintenant le format ne soient en contact avec le produit appliqué, en cours d'utilisation.

D'autres produits composites en formats peuvent rester totalement absorbants, et comprennent une ou deux couches externes disposées sur la nappe cent pour cent coton formée de trois couches, ces couches externes étant constituées d'un matériau textile tissé tel que la gaze. Ces produits sont utilisés comme compresses, par exemple pour le traitement des plaies.

Les produits composites sont réalisés sur des machines de découpe rotative.

On a réalisé des échantillons à l'échelle pilote sur une machine commercialisée par K. FASSBIND-LUDWIG AND CO. AG sous la marque FALU®.

Cette machine se compose d'un outil cylindrique supportant des moyens de découpe ou couteaux correspondant au format de découpe souhaité, qui vient s'appliquer contre une surface lisse d'un rouleau pour réaliser la découpe selon le format souhaité.

En amont de la découpe, un autre cylindre de calandrage et gaufrage à froid ou à chaud peut être prévu, venant au contact de l'outil cylindrique afin de réaliser des motifs sur la nappe alimentée sur l'outil cylindrique. Ici, on alimente en surperposition la nappe et la ou les couches externes. Au moment de la découpe, la ou les couches externes constituées au moins partiellement de fibres cellulosiques, différentes des couches formant la nappe cent pour cent coton, se lient à cette nappe par leurs périphéries. Les produits finis présentent une très nette ligne continue de sertissage.

Cette liaison mécanique des bords résulte de la nature cellulosique des fibres formant au moins partiellement la ou les couches externes et formant la nappe de coton, et du fait que ces fibres ou les matériaux formant la nappe et les couches externes ne sont pas ensimés.

A l'échelle industrielle, on utilise une machine de découpe rotative du type de celle commercialisée par la société SCHOBER.

On a mesuré la résistance à la délamination de produits composites sous forme de rondelles découpées sur la machine de marque FALU® décrite précédemment.

On a réalisé une rondelle selon l'invention (E), cent pour cent coton. c'est-à-dire non composite, de 200 g/m² et des rondelles composites à partir de la nappe formant la rondelle cent pour cent coton décrite ci-dessus.

Les rondelles composites (F), (G) et (H) comprennent une couche externe respectivement constituée d'une fine feuille de "tissue" ou papier, d'une feuille de gaze de coton, et d'une feuille de nontissé comprenant des fibres de coton et de viscose, et d'un grammage de 60 g/m².

La méthode de mesure de la résistance à la délamination consiste à :
- appliquer un film adhésif symétriquement sur chacune des faces de la rondelle, la surface d'un adhésif dépassant de chacune des faces de la rondelle,
- placer chaque partie d'adhésif dépassant de chacune des faces de la rondelle, entre les machoires d'un dynamomètre, et
- mesurer la force nécessaire à la séparation des couches.

Les résultats rapportés dans le tableau ci-après sont des intervalles de valeurs mesurées sur plusieurs rondelles selon l'invention cent pour cent coton (E), et composites (F) à (H). La résistance à la délamination est exprimée en centiNewton.

| | | | | |
|---|---|---|---|---|
| Rondelles | E | F | G | H |
| Force (cN) | 10 à 15 | 20 à 30 | 15 à 20 | 15 à 25 |

Il ressort du tableau qui précède que les rondelles composites ont une résistance à la délamination inattendue et bien améliorée par rapport à une rondelle selon l'invention non composite obtenue également après découpe sur une machine de type rotatif.

Ces nouveaux produits ont un aspect particulièrement bien fini et permettent à l'utilisateur de les manipuler par exemple sur la face externe en matériau différent de celui de la nappe de coton, sans toucher cette dernière, c'est-à-dire la face absorbante.

L'invention comprend tous les moyens équivalents ou supplémentaires à la portée de l'homme du métier, qui ne sont pas décrits précédemment.

## Revendications

1. Nappe de coton hydrophile (1) constituée à cent pour cent de fibres de coton, comprenant trois couches de coton, deux couches extérieures et une couche centrale, caractérisée en ce que les couches extérieures (3) et (4) entourant la couche centrale (2) sont des voiles de carde, en ce que la couche centrale (2) comprend des fibres de coton stratifiées orientées de manière sensiblement oblique entre les deux plans formés par les couches extérieures et en ce que la nappe (1) est obtenue par un procédé en ligne continue, consistant essentiellement à superposer les trois couches et à leur faire subir un traitement de débouillissage, un traitement de blanchiment puis un rinçage, conférant aux couches une très bonne cohésion entre elles.

2. Nappe selon la revendication 1, caractérisée en ce que les voiles de carde précités sont sensiblement isotropes et obtenus de préférence au moyen d'une carde du type pèle-mêle.

3. Nappe selon la revendication 1 ou 2, caractérisée en ce que la couche centrale (2) précitée est obtenue par voie pneumatique, au moyen d'une machine du type Rando.

4. Nappe selon l'une des revendications précédentes, caractérisée en ce que chaque couche extérieure (3) et (4) a un grammage compris dans l'intervalle allant d'environ 5 à environ 80 g/m², et de préférence d'environ 15 à environ 30 g/m².

5. Nappe selon l'une des revendications précédentes, caractérisée en ce que la couche centrale (2) a un grammage compris dans l'intervalle allant d'environ 60 à environ 400 g/m².

6. Nappe selon l'une des revendications précédentes, caractérisée en ce qu'elle est sous une forme sertie après découpe et présente des bords (6) de très faible épaisseur par exemple d'environ 0,2 millimètre pour une nappe de 2 millimètres d'épaisseur.

7. Nappe selon l'une des revendications précédentes, caractérisée en ce qu'elle n'a subi aucun traitement d'ensimage.

8. Produit de coton hydrophile sous forme paquetée, caractérisé en ce qu'il est obtenu par découpe ou prédécoupe d'au moins une nappe selon l'une des revendications 1 à 7.

9. Produit de coton hydrophile en formats, caractérisé en ce qu'il est obtenu par découpe ou prédécoupe d'au moins une nappe selon l'une des revendications 1 à 7.

10. Produit selon la revendication 9, caractérisé en ce que ledit produit a une forme en rondelle, carré, ou tout autre polygone régulier de coton, et est destiné au démaquillage ou à la toilette des bébés.

11. Produit selon la revendication 9, caractérisé en ce que la nappe est prédécoupée de manière à délimiter des formats tels que des rectangles, maintenus entre eux par des points de fixation.

12. Produit selon la revendication 9, caractérisé en ce qu'il est composite, et comprend la nappe de coton hydrophile (1) selon l'une des revendications 1 à 8 et au moins une quatrième couche externe (11) constituée au moins partiellement de fibres cellulosiques et située sur l'une des faces de ladite nappe (1).

13. Produit selon la revendication 12, caractérisé en ce qu'il comprend de plus une cinquième couche (13) constituée au moins partiellement de fibres cellulosiques et identique ou différente de la quatrième couche précitée (11) et située sur l'autre face de ladite nappe (1).

14. Produit selon la revendication 12 ou 13, caractérisé en ce que la (ou les) couche(s) externe(s) (11, 13) précitée(s) n'est (ne sont) pas ensimée(s).

15. Produit selon l'une des revendications 12 à 14, caractérisé en ce qu'il est obtenu par découpe au moyen d'une machine de découpe mécanique rotative et est sous une forme sertie.

16. Produit selon l'une des revendications 12 à 15, caractérisé en ce qu'il a une forme en rondelle, carré, rectangle ou tout autre polygone régulier.

17. Produit selon l'une des revendications 12 à 16, caractérisé en ce que ledit produit comporte un motif de gaufrage.

18. Produit selon l'une des revendications 12 à 17, caractérisé en ce que ladite quatrième couche (11) est un matériau nontissé ou tissé à base de fibres cellulosiques.

19. Produit selon la revendication 18, caractérisé en ce que ladite quatrième couche (11) est une couche de gaze et en ce que ledit produit est utilisé comme compresse.

20. Produit selon l'une des revendications 12 à 18, caractérisé en ce que ladite quatrième couche (11) est une feuille de nontissé et en ce que ledit produit est utilisé notamment comme tampon oculaire ou cosmétique.

21. Produit selon l'une des revendications 12 à 17, caractérisé en ce que ladite quatrième couche (11) est une feuille de papier, le cas échéant, plastifiée, et en ce que ledit produit est utilisé comme tampon cosmétique.

22. Produit selon l'une des revendications 13 à 17, caractérisé en ce que les quatrième et cinquième couches sont des couches de gaze et en ce que ledit produit est utilisé comme compresse.

## Patentansprüche

1. Hydrophiles Baumwollvlies (1), das zu 100% aus Raumwollfasern besteht, mit drei Baumwollschichten, zwei äußeren Schichten und einer mittleren Schicht, dadurch gekennzeichnet, daß die äußeren Schichten (3, 4), die die mittlere Schicht (2) umgeben, Kardenbänder sind, daß die mittlere Schicht (2) geschichtete Baumwollfasern aufweist, die im wesentlichen schräg zwischen den beiden von den äußeren Schichten gebildeten Ebenen aus gerichtet sind, und daß das Vlies (1) durch ein kontinuierliches Online-Verfahren erhalten wird, welches im wesentlichen darin besteht, daß die drei Schichten übereinandergelegt und einer Abbrühbehandlung, einer Bleichbehandlung und dann einem Spülvorgang unterzogen werden, was den Schichten einen sehr guten Zusammenhalt verleiht.

2. Vlies nach Anspruch 1, dadurch gekennzeichnet, daß die Kardenbänder im wesentlichen isotrop sind und Vorzugsweise mittels einer Deckelkarde (pêle-mêle) erhalten werden.

3. Vlies nach Anspruch 1 oder 2, dadurch gekennzeichnet, daß die mittlere Schicht (2) auf pneumatischem Weg mittels einer Maschine vom Typ Rando erhalten wird.

4. Vlies nach einem der vorhergehenden Ansprüche, dadurch gekennzeichnet, daß jede äußere Schicht (3,4) ein Gewicht in einem Bereich von ungefähr 5 bis ungefähr 80 g/m² und vorzugsweise ungefähr 15 bis 30 g/m² hat.

5. Vlies nach einem der vorhergehenden Ansprüche, dadurch gekennzeichnet, daß die mittlere Schicht (2) ein Gewicht in einem Bereich von ungefähr 60 bis ungefähr 400 g/m² hat.

6. Vlies nach einem der vorhergehenden Ansprüche, dadurch gekennzeichnet, daß es nach dem Schneiden eine gefalzte Form hat und Ränder (6) sehr geringer Dicke von beispielsweise ungefähr 0,2 mm für ein Vlies einer Dicke von 2 mm aufweist.

7. Vlies nach einem der vorhergehenden Ansprüche, dadurch gekennzeichnet, daß es keiner Schlichtebehandlung ausgesetzt wird.

8. Hydrophiles Baumwollprodukt in paketierter Form, dadurch gekennzeichnet, daß es durch Zuschneiden oder Vorschneiden mindestens eines Vlieses gemäß einem der Ansprüche 1 bis 7 erhalten wird.

9. Formatiertes hydrophiles Baumwollprodukt, dadurch gekennzeichnet, daß es durch Schneiden oder Vorschneiden mindestens eines Vlieses nach einem der Ansprüche 1 bis 7 erhalten wird.

10. Produkt nach Anspruch 9, dadurch gekennzeichnet, daß das Produkt die Form einer runden Scheibe, eines Quadrates oder irgendeines anderen gleichmäßigen Polygons aus Baumwolle hat und zum Abschminken oder zur Babypflege dient.

11. Produkt nach Anspruch 9, dadurch gekennzeichnet, daß das Vlies in Formen wie z.B. Rechtecke vorgeschnitten ist, welche durch Befestigungspunkte aneinander gehalten werden.

12. Produkt nach Anspruch 9, dadurch gekennzeichnet, daß es ein Mischprodukt ist und das hydrophile Baumwollvlies (1) nach einem der Ansprüche 1 bis 8 und mindestens eine vierte äußere Schicht (11) aufweist, die mindestens teilweise aus Zellulosefasern besteht und aufeiner der Seiten des Vlieses (1) angeordnet ist.

13. Produkt nach Anspruch 12, dadurch gekennzeichnet, daß es eine fünfte Schicht (13) aufweist, die mindestens teilweise von Zellulosefasern gebildet wird und identisch oder unterschiedlich zu der vierten Schicht (1 1) ist und auf der anderen Seite des Vlieses (1) angeordnet ist.

14. Produkt nach Anspruch 12 oder 13, dadurch gekennzeichnet, daß die äußere Schicht oder äußeren Schichten (11,13) keiner Schlichtebehandlung ausgesetzt ist bzw. sind.

15. Produkt nach einem der Ansprüche 12 bis 14, dadurch gekennzeichnet, daß es durch Zuschneiden mittels mindestens einer mechanischen Rotationsschneidmaschine erhalten wird und eine gefalzte Form hat.

16. Produkt nach einem der Ansprüche 12 bis 15, dadurch gekennzeichnet, daß es die Form einer runden Scheibe, eines Quadrats, eines Rechtecks oder irgendeines anderen gleichmäßigen Polygons hat.

17. Produkt nach einem der Ansprüche 12 bis 16, dadurch gekennzeichnet, daß das Produkt ein Gaufrier-Motiv aufweist.

18. Produkt nach einem der Ansprüche 12 bis 17, dadurch gekennzeichnet, daß die vierte Schicht (11) aus einem nicht gewebten oder gewebten Material auf Zellulosefaserbasis besteht.

19. Produkt nach Anspruch 18, dadurch gekennzeichnet, daß die vierte Schicht (11) eine Gazeschicht ist und daß das Produkt als Kompresse dient.

20. Produkt nach einem der Ansprüche 12 bis 18, dadurch gekennzeichnet, daß die vierte Schicht (11) ein nicht gewebtes Band ist und daß das Produkt insbesondere als Augentampon oder kosmetisches Tampon dient.

21. Produkt nach einem der Ansprüche 12 bis 17, dadurch gekennzeichnet, daß die vierte Schicht (11) ein Blatt aus gegebenenfalls plastifiziertem Papier ist und daß das Produkt als kosmetisches Tampon dient.

22. Produkt nach einem der Ansprüche 13 bis 17, dadurch gekennzeichnet, daß die vierte und fünfte Schicht Gazeschichten sind und daß das Produkt als Kompresse dient.

## Claims

1. Lap of hydrophilic cotton (1) composed one hundred per cent of cotton fibres, comprising three cotton layers, two exterior layers and one central layer,
characterised in that the exterior layers (3) and (4) surrounding the central layer (2) are card webs, in that the central layer (2) comprises laminated cotton fibres oriented substantially obliquely between the two planes formed by the exterior layers and in that the lap (1) is obtained by a process in a continuous line, consisting essentially in superimposing the three layers and in subjecting them to a scouring treatment, a bleaching treatment and then rinsing, imparting to the layers a very good mutual cohesion.

2. Lap according to claim 1, characterised in that the above-mentioned card webs are substantially isotropic and obtained preferably by means of a card of the pêle-mêle type.

3. Lap according to claim 1 or 2, characterised in that the above-mentioned central layer (2) is obtained by pneumatic means, by means of a machine of the Rando type.

4. Lap according to one of the preceding claims, characterised in that each exterior layer (3) and (4) has a weight lying in the range extending from about 5 to about 80 g/m², and preferably from about 15 to about 30 g/m².

5. Lap according to one of the preceding claims, characterised in that the central layer (2) has a weight lying in the range extending from about 60 to about 400 g/m².

6. Lap according to one of the preceding claims, characterised in that it is in a crimped form after cutting and has edges (6) of very reduced thickness, for example of about 0.2 millimetre, for a web 2 millimetres thick.

7. Lap according to one of the preceding claims, characterised in that it has not undergone any oiling treatment.

8. Hydrophilic cotton product in packaged form,
characterised in that it is obtained by the cutting or pre-cutting of at least one lap according to one of claims 1 to 7.

9. Hydrophilic cotton product in formats, characterised in that it is obtained by the cutting or pre-cutting of at least one lap according to one of claims 1 to 7.

10. Product according to claim 9, characterised in that said product is shaped as a washer, a square or any other regular polygon of cotton, and is intended for make-up removal or baby care.

11. Product according to claim 9, characterised in that the lap is pre-cut so as to delimit formats such as rectangles which are supported mutually by fixing points.

12. Product according to claim 9, characterised in that it is composite, and comprises the hydrophilic cotton lap (1) according to one of claims 1 to 8 and at least one fourth external layer (11) composed at least partially of cellulosic fibres and situated on one of the faces of said lap (1).

13. Product according to claim 12, characterised in that it comprises in addition a fifth layer (13) composed at least partially of cellulosic fibres and identical to or different from the aforementioned fourth layer (11) and situated on the other face of said lap (1).

14. Product according to claim 12 or 13, characterised in that the aforementioned external layer or layers (11, 13) is (are) not oiled.

15. Product according to one of claims 12 to 14,
characterised in that it is obtained by cutting by means of a rotary mechanical cutting machine and is in a crimped form.

16. Product according to one of claims 12 to 15,
characterised in that it is shaped as a washer, a square, a rectangle or any other regular polygon.

17. Product according to one of claims 12 to 16,
characterised in that said product exhibits an embossed pattern.

18. Product according to one of claims 12 to 17,
characterised in that said fourth layer (11) is a non-woven or woven material based on cellulosic fibres.

19. Product according to claim 18, characterised in that said fourth layer (11) is a layer of gauze and in that said product is used as a compress.

20. Product according to one of claims 12 to 18,
characterised in that said fourth layer (11) is a sheet of non-woven and in that said product is used in particular as an eye swab or make-up pad.

21. Product according to one of claims 12 to 17,
characterised in that said fourth layer (11) is a sheet of paper, plasticised in certain cases, and in that said product is used as a make-up pad.

22. Product according to one of claims 13 to 17,
characterised in that the fourth and fifth layers are layers of gauze and in that said product is used as a compress.
